(19) Europäisches Patentamt
European Patent Office
Office européen des brevets

(11) EP 3 053 649 A2

(12) **EUROPÄISCHE PATENTANMELDUNG**

(43) Veröffentlichungstag:
**10.08.2016 Patentblatt 2016/32**

(51) Int Cl.:
***B01J 35/10*** (2006.01) ***B01J 31/20*** (2006.01)
***C07C 45/50*** (2006.01) *B01J 21/08* (2006.01)

(21) Anmeldenummer: **16152950.8**

(22) Anmeldetag: **27.01.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(30) Priorität: **29.01.2015 DE 102015201560**

(71) Anmelder: **Evonik Degussa GmbH**
**45128 Essen (DE)**

(72) Erfinder:
- **HAHN, Hanna**
**47199 Duisburg-Baerl (DE)**
- **DYBALLA, Katrin Marie**
**45657 Recklinghausen (DE)**
- **FRANKE, Robert**
**45772 Marl (DE)**
- **FRIDAG, Dirk**
**45721 Haltern am See (DE)**
- **WALTER, Simon**
**79595 Rümmingen (DE)**
- **HAUMANN, Marco**
**91235 Velden (DE)**
- **WASSERSCHEID, Peter**
**91054 Erlangen (DE)**

(54) **SILP (GETRÄGERTE IONISCHE FLÜSSIGPHASE) -KATALYSATOR ZUR HYDROFORMYLIERUNG VON OLEFINEN MIT SYNTHESEGAS**

(57) Die Erfindung betrifft einen SILP-Katalysator zur Hydroformylierung von Olefinen mit Synthesegas sowie ein Verfahren zur Hydroformylierung von Olefinen mit Synthesegas unter Verwendung des Katalysators.

Ihr liegt die Aufgabe zu Grunde, ein Modell zu entwickeln, anhand dessen der Transport und die Reaktion in SILP-Katalysatoren beschrieben werden kann. Ausgehend davon sollen alternative Katalysatoren für die SILP-Katalyse bereitgestellt werden.

Gelöst wird diese Aufgabe von einem SILP-Katalysator, der zumindest ein kugelförmiges Katalysatorpellet aufweist und der einen besonderen SILP-Modul einhält.

Fig. 1

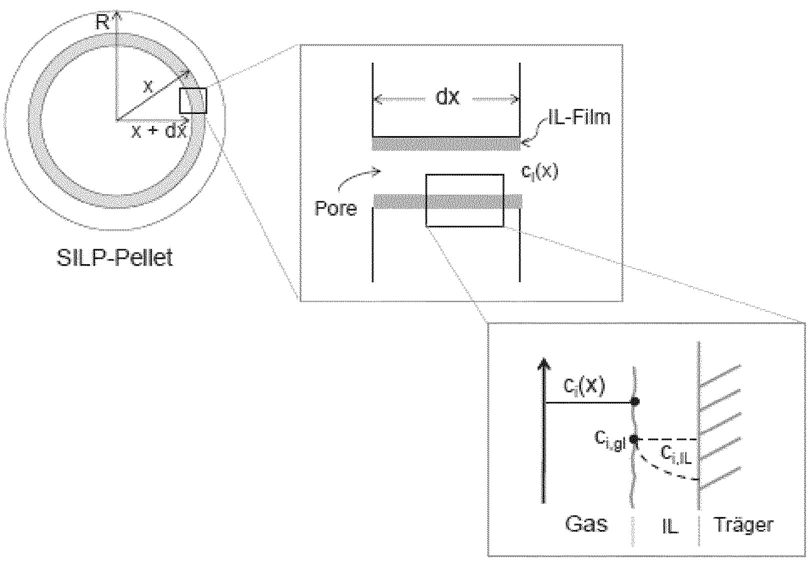


EP 3 053 649 A2

Printed by Jouve, 75001 PARIS (FR)

**Beschreibung**

Die vorliegende Erfindung betrifft einen SILP-Katalysator zur Hydroformylierung von Olefinen mit Synthesegas sowie ein Verfahren zur Hydroformylierung von Olefinen mit Synthesegas unter Verwendung des Katalysators.

Die Umsetzung von Olefinverbindungen mit einer Mischung aus Kohlenstoffmonoxid und Wasserstoff in Gegenwart eines Katalysators zu Aldehyden ist als Hydroformylierung bzw. Oxosynthese oder Roelen-Reaktion bekannt (Abbildung 1):

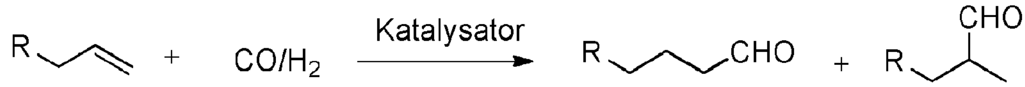


Abb. 1: Reaktionsschema der Hydroformylierung von Olefinen

Homogene Katalysatorkomplexe für die Hydroformylierung weisen in der Regel die allgemeine Struktur $H_x M_y(CO)_z L_n$ auf, wobei M ein Übergangsmetallatom ist, das Metallcarbonylhydride ausbilden kann und L ein Ligand bzw. mehrere Liganden sind.

Bevorzugte Übergangsmetalle für die Verwendung in den vorgenannten Katalysatorkomplexen sind Kobalt und Rhodium, weniger bevorzugt sind Ruthenium, Iridium, Palladium, Platin und Eisen. Angefangen mit der Entdeckung Phosphan-modifizierter Katalysatoren in der Hydroformylierung im Jahre 1968 sind heute Ligand-modifizierte Kobalt- und Rhodiumkatalysatoren Stand der Technik.

Für die industrielle Hydroformylierung gibt es verschiedene Verfahren, wobei das Ruhrchemie/Rhône-Poulenc-Verfahren einen Meilenstein in der Entwicklung der industriellen Hydroformylierung darstellt. In diesem Verfahren besteht das Katalysatorsystem aus Rhodium und einem wasserlöslichen Liganden und ist in einer wässrigen Phase gelöst, wobei das Edukt-Produkt-Gemisch eine zweite flüssige Phase bildet. Nach der Vermischung beider Phasen durch Rühren und der Durchströmung von Synthesegas und Olefin, falls dieses gasförmig vorliegt, wird das Edukt-Produkt-Gemisch durch Phasentrennung von dem Katalysatorsystem abgetrennt und destillativ aufgearbeitet.

Nachteilig an diesem Verfahren ist, dass Rhodiumverluste durch Auslaugung nicht vermeidbar sind, was angesichts der Rhodiumpreise problematisch ist. Weiter ist nachteilig, dass das Spektrum an einsetzbaren kurzkettigen Olefinen begrenzt ist. Denn die Löslichkeit der Olefine in der wässerigen Katalysatorphase nimmt mit zunehmender Kettenlänge ab, wodurch sich die Reaktionsgeschwindigkeit reduziert. Dieser Effekt kann nur bedingt durch intensives Rühren kompensiert werden, sodass eine wirtschaftliche Durchführung des Prozesses für > C4 nicht gewährleistet werden kann.

Bei der Entwicklung von Alternativverfahren wurde insbesondere angesichts der sehr hohen Rhodiumpreise die Idee verfolgt, die bislang homogen in der Reaktionsmischung vorliegenden Rhodium-basierten Katalysatorsysteme zu immobilisieren. Dabei wird das "einfache" Abtrennen der Katalysatorspezies von der Edukt/Produkt-Phase ohne ein zusätzliches Trennverfahren allgemein als Immobilisierung bezeichnet. Immobilisierungskonzepte zielen darauf ab, die katalytisch aktive Spezies in einer anderen Phase als die Edukte/Produkte zu halten. Abbildung 2 enthält eine Übersicht über Immobilisierungskonzepte homogener Katalysatoren.

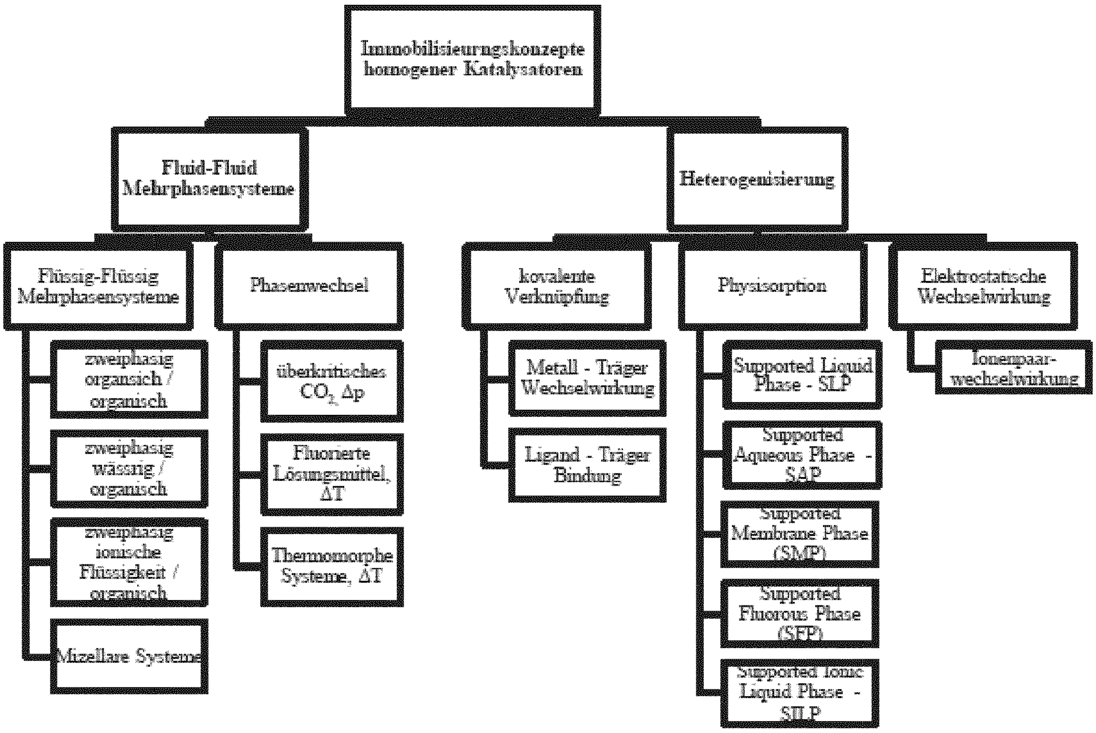


Abb. 2: Immobilisierungskonzepte für homogene Katalysatoren

In der Literatur existieren zahlreiche Ansätze zur Immobilisierung von homogenen Katalysatoren (siehe z.B. E. Lindner, T. Schneller, F. Auer, H. A. Mayer, Chemistry in Interphases- A New Approach to Organometallic Syntheses and Catalysis, Angew. Chem., Int. Ed. 1999, 38, 2154-2174 und D. J. Cole-Hamilton, R. P. Tooze, Catalyst Separation, Recovery and Recyling, Springer, 2006). Homogene Katalysatorkomplexe können in eine Polymermatrix eingeschlossen werden, über ionische oder kovalente Bindungen mit dem Support verankert werden, über Wasserstoffbrückenbindungen mit dem Träger verknüpft werden oder in einem dünnen physisorbierten Film eines Fluides gelöst auf dem Träger vorliegen. All diese Möglichkeiten haben ihre Vor- und Nachteile. Katalysator-Leaching stellt außer bei der ionischen oder kovalenten Verankerung von Katalysator und Träger das größte Problem dar. Für eine kovalente Verknüpfung muss der Ligand speziell hergestellt werden und Leaching wird nur bei hinreichend starker Verknüpfung vermieden. Für eine ionische Verknüpfung muss der Katalysatorkomplex ebenfalls eine Ladung tragen, jedoch kann die Stabilität durch Gegenionen beeinflusst werden, die bei Reaktionen mit eventueller Salzbildung entstehen. Bei Verknüpfung über Wasserstoffbrückenbindungen steht der Ligand in Konkurrenz zu den Edukten, Produkten und Lösungsmitteln.

Neben dem Supported-Aqueous-Phase (SAP) Konzept, das jedoch für hydrolyseempfindliche Liganden ungeeignet ist, stellt das Supported-Liquid-Phase (SLP) Konzept ein weiteres Konzept zur Heterogenisierung von homogenen Katalysatorkomplexen dar. Supported Liquid Phase (SLP) Katalysatoren basieren auf der Physisorption eines Lösungsmittels auf der Oberfläche eines porösen Trägers, wobei in dem Lösungsmittel ein homogener Katalysatorkomplex gelöst ist. Für die Hydroformulierung werden unter anderem geschmolzene Salze wie z.B. Triphenylphosphan (TPP) als flüssige Phase eingesetzt. TPP dient hierbei zum einen als Lösungsmittel für den Katalysatorkomplex, und zum anderen als Ligand und wird deshalb in hohem Überschuss eingesetzt. Nachteilig an einem sehr großen Ligandenüberschuss ist jedoch die Bildung verschiedener Übergangsmetallkomplexe, die eine Unterdrückung der katalytischen Aktivität bewirken können.

Ein weiteres Konzept, bei dem heterogenisierte Katalysatorkomplexe eingesetzt werden, ist das Supported Ionic Liquid Phase (SILP) Konzept. Ein Supported Ionic Liquid Phase (SILP)-Katalysator besteht aus einem katalytisch aktiven Metallkomplex, einer ionischen Flüssigkeit und einem Trägermaterial. Der Katalysatorkomplex ist in der ionischen Flüssigkeit gelöst und diese liegt im Idealfall als dünner Film an den Porenwänden des Trägers immobilisiert vor. Makroskopisch wird ein heterogener Katalysator erhalten, dessen Aktivität und Selektivität analog zur homogenen Katalyse bei vergleichbaren Reaktionsbedingungen sind.

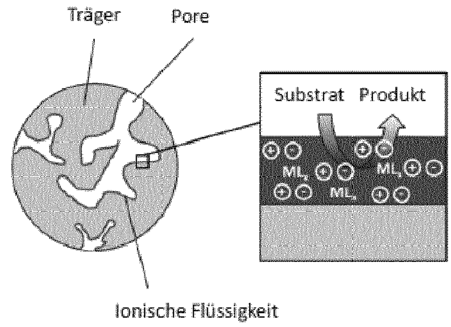


Abb. 3: Konzept der Supported Ionic Liquid Phase (SILP) Katalyse [Quelle: R. Fehrmann, A. Riisager, M. Haumann, *Supported Ionic Liquids: Fundamentals and Applications*, Wiley-VCH, 2014].

Gegenüber dem bekannten Stand der Technik ist es die Aufgabe der vorliegenden Erfindung, ein Modell zu entwickeln, anhand dessen der Transport und die Reaktion in SILP-Katalysatoren beschrieben werden kann. Ausgehend davon sollen alternative Katalysatoren für die SILP-Katalyse, insbesondere für die SILP-katalysierte Hydroformylierung von Olefinen mit Synthesegas, sowie ein Verfahren zur SILP-katalysierten Hydroformylierung von Olefinen mit Synthesegas unter Verwendung dieser Katalysatoren bereitgestellt werden.

Die Erfindung löst diese Aufgabe durch einen SILP-Katalysator sowie durch ein Verfahren zur Hydroformylierung von Olefinen mit Synthesegas unter Verwendung eines SILP-Katalysators mit den Merkmalen der Ansprüche, und insbesondere durch einen SILP-Katalysator, der zumindest ein kugelförmiges Katalysatorpellet mit dem Radius R aufweist und der das folgende SILP-Modul einhält:

$$\frac{c_i}{c_{i,S}} = \frac{R}{x}\,\frac{\sinh\left(\phi_{SILP}\frac{x}{R}\right)}{\sinh(\phi_{SILP})} \qquad \text{(Gl. 1)}$$

wobei $\varphi_{SILF}$ eine dimensionslose Kennzahl für das kugelförmige Katalysatorpellet darstellt, die den Stofftransport in der Pore, über die Phasengrenzfläche zwischen Gas und ionischer Flüssigkeit und in der ionischen Flüssigkeit zur Reaktion ins Verhältnis setzt,
wobei $c_i$ die Konzentration einer Komponente i und $c_{i,s}$ deren Konzentration an der Oberfläche des SILP-Katalysatorpellets, und x der Ort ist.

Das nachstehend beschriebene Modell, das den Stofftransport einer gasförmigen Komponente und die Reaktion in einem SILP-Katalysator beschreibt, ist an das literaturbekannte Modul von Thiele und an das Zweifilmmodel nach Lewis und Whitman angelehnt (siehe: E. W. Thiele, 'Relation between Catalytic Activity and Size of Particle', Ind. Eng. Chem. 1939, 31, 916 - 920 und W. K. Lewis, W. G. Whitman, 'Principles of Gas Absorption', Ind. Eng. Chem. 1924, 16, 1215-1220).

Die Aktivität eines SILP-Katalysators wird nicht nur durch die Art der homogenen Edelmetallkomplexe beeinflusst; die Verteilung der Komplexe und der ionischen Flüssigkeit (IL) spielt ebenfalls eine Rolle. Des Weiteren haben die Porenradienverteilung, das Benetzungsverhalten der ionischen Flüssigkeit, der Anteil der IL im Porennetzwerk, die Löslichkeit der Substrate und Produkte in der IL, die Wechselwirkungen von Edelmetallkomplex und Träger bzw. Komplex und IL und optional auch die Präparation des SILP-Katalysators einen Einfluss auf die Aktivität. Untersuchungen zu den Wechselwirkungen der ionischen Flüssigkeit [BMIM][NTf2] mit einer idealen planaren Oberfläche eines Alumina-Trägers zeigten eine kontinuierliche Filmbildung im Nanometerbereich (siehe: M. Sobota, I. Nikiforidis, W. Hieringer, N. Paape, M. Happel, H.-P. Steinruck, A. Gorling, P. Wasserscheid, M. Laurin, J. r. Libuda, 'Toward Ionic-Liquid-Based Model Catalysis: Growth, Orientation, Conformation, and Interaction Mechanism of the [Tf2N]-Anion in [BMIM][Tf2N] Thin Films on a Well-Ordered Alumina Surface', Langmuir 2010, 26, 7199-7207). Haumann et al. konnten mittels Festkörper-NMR-Messungen zeigen, dass sich für Beladungen unter 10 Vol.-% Inseln an ionischer Flüssigkeit auf realen Porenwänden ausbilden (siehe: M. Haumann, A. Schönweiz, H. Breitzke, G. Buntkowsky, S. Werner, N. Szesni, 'Solid-State NMR Investigations of Supported Ionic Liquid Phase Water-Gas Shift Catalysts: Ionic Liquid Film Distribution vs. Catalyst Performance', Chem. Eng. Technol. 2012, 35, 1421-1426. Mit höherer Beladung wurde kein Signal für freie Silanolgruppen an der Oberfläche des Trägers detektiert. Daraus lässt sich schließen, dass sich ein monomolekularer Film auf der Oberfläche des Trägers bildet, aber nicht, ob ein kontinuierliches homogenes Anwachsen des Films stattfindet. An der

Phasengrenzfläche Gas/IL konnte nachgewiesen werden, dass die Ausrichtung der Alkylkette am Kation der IL die Struktur der Phasengrenzfläche und somit die Löslichkeit und den Phasenübergang beeinflussen. Das Zusammenspiel all dieser Effekte ist jedoch sehr kompliziert und bis heute nicht im Detail geklärt. Daher werden folgende Annahmen für das Modell getroffen:

- Das Fluid ist gasförmig oder flüssig, keine Mischung aus zwei Phasen.
- Das Katalysatorpellet ist isotherm, Temperatureffekte werden nicht berücksichtigt.
- Der Träger wird als inert betrachtet und Diffusion der reagierenden Gase und Produkte findet nur in der Pore statt.
- Die Poren sind untereinander verbunden.
- Filmdiffusion durch die äußere Grenzschicht des Pellets ist im Vergleich zur Diffusion in der Pore sehr schnell.
- Stofftransport findet ausschließlich aufgrund von Diffusion statt.
- Die Reaktion ist erster Ordnung und die Rückreaktion wird nicht berücksichtigt.

In Figur 1 ist eine Veranschaulichung des Modells geben. Den Bilanzraum bildet ein poröses, kugelförmiges SILP-Katalysatorpellet. Es werden der Stofftransport in einer mit ionischer Flüssigkeit gefüllten Pore und der Stofftransport über die Gas/IL Phasengrenzfläche mit anschließender Reaktion beschrieben.

In einer ersten Näherung kann angenommen werden, dass kein Konzentrationsgradient im IL-Film vorliegt. Diese Annahme ist für kleine Beladungen, bei denen die Filmdicke im Nanometerbereich liegt, sicherlich zutreffend. Ionische Flüssigkeiten besitzen im Vergleich zu organischen Lösungsmitteln eine zwei- bis dreimal höhere Viskosität. Eine höhere Viskosität resultiert in einem kleineren Diffusionskoeffizienten in der flüssigen Phase. Für Kohlenstoffdioxid, das eine mit organischen Lösungsmitteln vergleichbar hohe Löslichkeit in ILs besitzt, ist der Diffusionskoeffizient in [EMIM][NTf2] im Vergleich zu Toluol um das zehnfache reduziert. In dem Modell werden daher drei Fälle unterschieden (vgl. Fig. 2: Konzentrationsverlauf im IL-Film in Abhängigkeit von Stofftransport und Reaktion nach Lewis und Whitman, Quelle: W. K. Lewis, W. G. Whitman, 'Principles of Gas Absorption', Ind. Eng. Chem. 1924, 16, 1215-1220.):

Fall I: Reaktion und Stofftransport laufen simultan im IL-Film ab.
Fall II: Die Reaktion ist im Vergleich zum Stofftransport langsam.
Fall III: Stofftransport ist wesentlich schneller als die Reaktion aufgrund des dünnen IL-Films.

Ausgangsformel bildet die allgemeine Stoffbilanz eines Stoffes i, die um einen Term zur Beschreibung des Phasenübergangs Gas/IL erweitert wurde:

$$\frac{\partial c_i}{\partial t} = -div\,(c_i \vec{u}) + div\,(D_i\; grad\; c_i) + R_i \pm a\,\beta_i\left(c_i - c_{i,gl}\right) \qquad \text{(Gl. 2)}$$

In den Annahmen wird Strömung aufgrund von Konvektion bereits ausgeschlossen. Daraus resultiert, dass die Geschwindigkeit u = 0 ist. Der Dispersionskoeffizient $D_i$ in der Gasphase ist ortsunabhängig und wird im Folgenden als effektiver Diffusionskoeffizient $D_{eff}$ bezeichnet. Unter Berücksichtigung nur einer Raumrichtung resultiert:

$$\frac{\partial c_i}{\partial t} = D_{eff}\, d\frac{dc}{dx} + R_i + a\beta_i(c_{i,} - c_{i,gl}) \qquad \text{(Gl. 3)}$$

Für ein kugelförmiges Pellet vom Radius R ergibt sich die Stoffbilanz für eine volumenbeständige und irreversible Reaktion unter den eingangs formulierten Annahmen aus der allgemeinen Stoffbilanz für einphasige Reaktionssysteme. Mit $\partial c_i / \partial t = 0$ (stationäre Verhältnisse) und $R_i = v_{ij} r_j = -1kc_{i,n}$ lautet die Stoffbilanz:

$$D_{eff}\left(\frac{d^2 c_i}{dx^2} + \frac{2}{x}\frac{dc_i}{dx}\right) - \left(-kc_{i,IL} \pm a\,\beta_i(c_{i,IL} - c_{i,gl})\right) = 0 \qquad \text{(Gl. 4)}$$

Der effektive Diffusionskoeffizient der gasförmigen Komponente in der leeren Pore wird über folgende Beziehung beschrieben.

$$D_{eff}^{0} = \frac{D_{i,g}\,\varepsilon^{0}}{\tau^{0}} \;/\; \mathrm{m^2\,s^{-1}} \qquad \text{(Gl. 5)}$$

Aus Gleichung 5 ist ersichtlich, dass der effektive Diffusionskoeffizient von der Porosität und Tortuosität des Trägers abhängt. Beide Faktoren werden in einem SILP-Katalysator zusätzlich durch die Beladung $\alpha$ an ionischer Flüssigkeit beeinflusst. Bei der Beladung $\alpha$ handelt es sich um eine volumenbezogene Größe. Daher kann folgende Beziehung für die Porosität angegeben werden:

$$\varepsilon = \varepsilon_P \;(1-\alpha) \qquad \text{(Gl. 6)}$$

Die Tortuosität ist ein Konzept, das das Verhältnis von mittlerer Porenlänge ($L_e$) zu der Länge (L) beschreibt, entlang derer der Diffusionsstrom definiert ist.

$$\tau = L_e/L \qquad \text{(Gl. 7)}$$

In der Literatur existieren zahlreiche Korrelationen, die einen Zusammenhang zwischen der Porosität und der Tortuosität herstellen. Folgender Zusammenhang erweist sich als besonders zutreffend:

$$\tau = 1 - p\ln\varepsilon \qquad \text{(Gl. 8)}$$

Der Parameter p stellt hier einen Anpassungsparameter dar, der durch physikalische Effekte, wie beispielsweise die Hygroskopizität des Trägers, modifiziert wird. In diesem Modell soll ebenfalls diese Beziehung zur Beschreibung des effektiven Diffusionskoeffizienten einfließen. Der Parameter p beschreibt in diesem Modell die Homogenität der IL-Filmbildung:

$$\tau = 1 - p\ln\varepsilon^{0}(1-\alpha), \qquad \text{(Gl. 9)}$$

mit p =1 (ideale Filmbildung), p $\to$ 0 (Inselbildung bzw. Fluten der Poren und Filmbildung).

Unter Berücksichtigung von Gleichung 6 und Gleichung 9 hängt der effektive Diffusionskoeffizient von der Beladung $\alpha$ wie folgt ab:

$$D_{eff} = \frac{D_{i,g}\,\varepsilon^{0}\,(1-\alpha)}{1-p\ln\varepsilon_P\;(1-\alpha)} \;/\; \mathrm{m^2\,s^{-1}} \qquad \text{(Gl. 10)}$$

Durch Einführen der Beziehungen:

$$H_{12} = c_i/c_{i,gl} > 1,\ \text{mit}\ H_{12} = \text{Henry-Koeffizient} / - , \qquad \text{(Gl. 11)}$$

Einführen dimensionsloser Größen:

$$y = x\,/\,R, \qquad \text{(Gl. 12)}$$

$$f = c_i/c_{i,S} \qquad \text{(Gl. 13)}$$

erhält man aus Gleichung 4

$$\frac{2}{y}\frac{df}{dy} + \frac{d^2 f}{dy^2} - \phi_{SILP} f = 0 \qquad \text{(Gl. 14)}$$

wobei $\varphi_{SILP}$ eine dimensionslose Kennzahl für ein kugelförmiges SILP-Partikel darstellt, die Stofftransport in der Pore, über die Gas/IL Phasengrenzfläche und in der IL zur Reaktion ins Verhältnis setzt. In Abhängigkeit der betrachteten Konzentrationsprofile in dem IL-Film konnten folgende Moduli $\varphi_{SILP}$ abgeleitet werden:

Tabelle 1: Modifizierte Thiele-Moduli für ein kugelförmiges SILP-Katalysatorpellet in Abhängigkeit des Stofftransportes im IL-Film

| Fall I | Fall II | Fall III |
|---|---|---|
| $\phi_{SILP,I} = R\sqrt{\frac{\sqrt{k\,D_i^{IL}}\,a}{D_{eff}\,H_{12}}}$ | $\phi_{SILP,II} = R\sqrt{\frac{2\,k\,\beta_i\,a}{D_{eff}\,H_{12}(k+\beta_i\,a)}}$ | $\phi_{SILP,III} = R\sqrt{\frac{k}{D_{eff}\,H_{12}}}$ |

Für die Stoffaustauschfläche wird folgende Beziehung aus einer geometrischen Abhängigkeit für eine ideale zylindrische Probe definiert:

$$a = a^0\sqrt{1-\alpha} \qquad \text{(Gl. 15)}$$

Mit den Randbedingungen

$$(I)\ y = 1 \Rightarrow f = 1 \qquad \text{(Gl. 16)}$$

$$(II)\ y = 0 \Rightarrow \frac{df}{dy} = 0 \quad \text{(Symmetriebedingung)} \qquad \text{(Gl. 17)}$$

folgt folgende Lösung aus Gleichung 14:

$$f = \frac{1}{y}\frac{\left(e^{\phi_{SILP}Y} - e^{-\phi_{SILP}Y}\right)}{\left(e^{\phi_{SILP}} - e^{-\phi_{SILP}}\right)} = \frac{1}{y}\frac{\sinh(\phi_{SILP}Y)}{\sinh(\phi_{SILP})} \qquad \text{(Gl. 18)}$$

Resubstitution von Gleichung 18 liefert das Verhältnis der Konzentration $c_i$ zu der Konzentration an der Pelletoberfläche $c_{i,s}$ in Abhängigkeit des Ortes x:

$$\frac{c_i}{c_{i,S}} = \frac{R}{x}\frac{\sinh\left(\phi_{SILP}\frac{x}{R}\right)}{\sinh(\phi_{SILP})} \qquad \text{(Gl. 19)}$$

Mit Hilfe von Gleichung 19 kann eine auf das Reaktionsvolumen $V_R$ bezogene effektive Reaktionsgeschwindigkeit $r_{eff}$ definiert werden. Aus dem Verhältnis von effektiver zur intrinsischen Reaktionsgeschwindigkeit ergibt sich der Katalysatorwirkungsgrad bzw. Porennutzungsgrad eines SILP-Pellets:

$$\eta = \frac{(r_{eff})_{V_R}}{r_{intr}} \quad \text{(Gl. 20)}$$

$$r_{eff,V_R} = \frac{1}{V_k} \int_0^R r(x) 4\pi x^2 dx \quad \text{(Gl. 21)}$$

$$r(x) = k_{V_{IL}} \cdot c_i \quad \text{(Gl. 22)}$$

$$V_K = \frac{4}{3}\pi R^3 \quad \text{(Gl. 23)}$$

$$\Rightarrow \eta_P = \frac{r_{eff}}{r} = \frac{3}{\phi_{SILP}} \left(\frac{1}{\tanh(\phi_{SILP})} - \frac{1}{\phi_{SILP}}\right) \quad \text{(Gl. 24)}$$

Analog zur Interpretation des Thiele Moduls führen Werte < 3 für die SILP-Moduli zu einem Wirkungsgrad nahe 100 %.

Im Folgenden werden die Moduli anhand einer Modellreaktion A $\rightarrow$ B diskutiert. Der Einfluss eines einzelnen Parameters in den Moduli ist dabei immer in Relation zu den anderen Parametern zu betrachten und nicht absolut.

Figur 3 stellt den Porennutzungsgrad als Funktion der erhaltenen modifizierten Thiele-Moduli gegen die Beladung mit ionischer Flüssigkeit dar. Für Fall III, in dem kein Konzentrationsgradient im IL-Film vorliegt, wird der höchste Porenwirkungsgrad erzielt. Für Fall I zeigt sich der kleinste Wirkungsgrad in Abhängigkeit der Beladung.

In den Verläufen spiegeln sich die getroffenen Annahmen der jeweiligen Fälle wieder. Ein starker Abfall der Konzentration im IL-Film hat einen erhöhten Stoffmengenstrom im Film zur Folge. Aus der Annahme resultiert, dass die Reaktion in der Flüssigphase schneller erfolgt als der Stoffmengenstrom in den Film. Der verlangsamte Stofftransport über die Gas/IL-Phasengrenzfläche im Verhältnis zur Reaktion führt unter Berücksichtigung des effektiven Diffusionskoeffizienten zu einer schlechteren Ausnutzung des SILP-Katalysatorpellets. Für eine Erhöhung des Wirkungsgrads müsste der effektive Diffusionskoeffizient durch die Wahl eines anderen Trägers bzw. Porengefüges vergrößert oder die Löslichkeit der Edukte in der ionischen Flüssigkeit drastisch erhöht werden.

Der Einfluss der Löslichkeit, ausgedrückt durch den Henry-Koeffizient, und der Porosität des Trägers ist für den Fall III in Figur 4 dargestellt.

Grundsätzlich zeigt sich, dass der Katalysatorwirkungsgrad mit zunehmender Beladung abnimmt. Dieses Verhalten ist mit dem sinkenden effektiven Diffusionskoeffizienten in der sich verengenden Pore verbunden. Der Grenzfall $\alpha = 1$ und $\alpha = 0$ ist in diesem Modell nicht berücksichtigt. Es kann in einigen Fällen theoretisch möglich sein, mit einer Beladung von $\alpha = 1$ einen höheren Wirkungsgrad als mit einer Beladung $\alpha < 1$ zu erzielen.

Figur 4a stellt den Wirkungsgrad eines SILP-Katalysatorpellets gegen die Beladung an ionischer Flüssigkeit in Abhängigkeit des Henry-Koeffizienten unter Bedingungen für das SILP Modul Typ III $\Phi_{SILP,III}$ dar. Der Henry-Koeffizient variiert im Bereich $H_{12}$ = 1-1000. Der Henry-Koeffizient stellt eine IL-spezifische Größe dar. Mit abnehmenden Werten für den Henry-Koeffizienten steigt die Löslichkeit des Substrates in der ionischen Flüssigkeit. Folglich reduziert sich der Katalysatorwirkungsgrad mit zunehmender Löslichkeit der Substrate, bei entsprechend kleinem effektiven Diffusionskoeffizienten.

Figur 4b stellt den Wirkungsgrad eines SILP-Katalysatorpellets gegen die Beladung an ionischer Flüssigkeit in Abhängigkeit der Porosität des Trägers unter Bedingungen für das SILP Modul Typ III $\Phi_{SILP,III}$ dar. Mit zunehmendem Porenvolumen sinkt der Katalysatorwirkungsgrad. Allerdings erhöht sich mit der Porosität ebenfalls die Stoffaustauschfläche, die eine schnelle Umsetzung begünstigt. Es sollte beachtet werden, dass die absolute Menge an ionischer Flüssigkeit zu unterschiedlichen Filmdicken in Abhängigkeit der Porosität führt. Daraus können letztendlich ähnliche Wirkungsgrade erreicht werden. Die optimale Porosität eines Trägers für die SILP-Katalyse wird somit von vielen Faktoren beeinflusst. Beispielsweise können für eine stark exotherme und schnell ablaufende Reaktion eine kleine Porosität und eine hohe Beladung zu einer verbesserten Abfuhr der Wärme aus dem Katalysatorbett führen.

Für den Fall, dass der Stofftransport über die Gas/IL-Phasengrenzfläche und eine schnelle Reaktion simultan ablaufen, wird das SILP-Modul $\Phi_{SILP,I}$ erhalten. In Figur 5 ist der Wirkungsgrad gegen die Beladung an ionischer Flüssigkeit in Abhängigkeit der Stoffaustauschfläche für das SILP-Modul $\Phi_{SILP,I}$ dargestellt.

Im Falle eines Konzentrationsgradienten im IL-Film ist die Stoffaustauschfläche von entscheidender Bedeutung für die Ausnutzung des Pellets. Für den Henry-Koeffizient wird ein konstanter Wert von $H_{12}$ = 10 in Figur 5 festgelegt. Die spezifische Stoffaustauschfläche wird im Bereich von $10^2$ $m^{-1}$ bis $10^{10}$ $m^{-1}$ variiert. Weiterhin wird angenommen, dass der IL-Film homogen über den Querschnitt einer zylindrischen Pore mit der Beladung anwächst. Eine große Stoffaustauschfläche begünstigt eine schnelle Umsetzung der Reaktanden. Für den Fall, dass ein teurer Katalysatorkomplex eingesetzt wird, führt eine zu große Austauschfläche zu einem schlechten Wirkungsgrad und letztendlich zu einer unökonomischen Ausnutzung des Katalysators. Im Bereich von Literaturwerten für den Diffusionskoeffizienten $D_{i,IL}$ von Gasen, wie beispielsweise Wasserstoff, Kohlenstoffdioxid, Ethen oder Propen, in ionischen Flüssigkeiten zeigen diese einen vernachlässigbaren Einfluss auf den Wirkungsgrad in Abhängigkeit der Beladung.

Fall II betrachtet Stofftransport und Reaktion getrennt voneinander, sodass neben dem Henry- und dem Diffusionskoeffizienten auch die formale "Grenzschichtdicke" $\delta$ in das Modell einfließen. Der Einfluss des Stoffübergangskoeffizienten $\beta$ auf den Wirkungsrad ist sehr gering und wird daher nicht näher beschrieben.

Alle drei Fälle zeigen, dass die Löslichkeit der Substrate und der effektive Diffusionskoeffizient den größten Einfluss auf den Wirkungsgrad haben. Eine Verbesserung der Diffusion in der Pore lässt sich entweder durch die Morphologie des Trägers oder durch eine Erhöhung des Diffusionskoeffizienten, beispielsweise durch Temperaturerhöhung bewirkt, bewerkstelligen.
Die Modelle zeigen weiterhin, dass eine Erhöhung der Löslichkeit zu einer Steigerung der Reaktionsgeschwindigkeit führt, aber nicht zwangsläufig zu einer besseren Ausnutzung des Pellets.

Erfindungsgemäße Katalysatoren wurden durch Versetzen poröser Trägermaterialien mit einer Lösung, die einen Katalysatorkomplex, ionische Flüssigkeit und Stabilisator enthielt, hergestellt. Dabei wurde bevorzugt Dichlormethan als Lösungsmittel eingesetzt. Das Abscheiden der Komponenten in dem Porennetzwerk des Trägers erfolgte bevorzugt durch Einengen am Rotationsverdampfer. Nach Entfernung des Lösungsmittels liegt der Katalysator als rieselfähiges Pulver vor.

Die Hydroformylierung wurde besonders bevorzugt in der Gasphase durchgeführt, wobei Synthesegas als Mischung von Kohlenstoffmonoxid (Linde AG, 3.7) und Wasserstoff (Linde AG, 5.0) eingesetzt wurde. Als Olefin wurden insbesondere kurz- bis mittelkettige ungesättigte Kohlenwasserstoffe, z.B. 1-Buten oder 2-Buten eingesetzt.

Bevorzugte Katalysatorkomplexe, die für die Synthese von SILP-Katalysatoren eingesetzt wurden, sind in der nachstehenden Tabelle 2 aufgeführt.

Tabelle 2: Übersicht über die eingesetzten Präkursoren und homogenen Katalysatoren

| Komplex | Abkürzung | Molekulargewicht $gmol^{-1}$ | CAS-Nummer |
|---|---|---|---|
| (Acetylacetonat)dicarbonylrho dium(I) | $Rh(CO)_2(acac)$ | 258,03 | 14874-82-9 |
| (Acetylacetonat)(1,5-cyclooctadien)rhodium(I) | Rh(acac)(COD) | 310,19 | 12245-39-5 |
| Tris(triphenylphosphin)rrhodiu m(I)chlorid | $RhCl(PPh_3)_3$ | 925,22 | 14694-95-2 |
| Rhodium(I)chlorid | $RhCl_3$ | 209,26 | 10049-07-7 |

Bevorzugte ionische Flüssigkeiten, die für die Synthese von SILP-Katalysatoren eingesetzt wurden, sind in der nachstehenden Tabelle 3 aufgeführt.

Tabelle 3: Stoffdaten der eingesetzten ionischen Flüssigkeiten

| Ionische Flüssigkeit | Abkürzung | Molar Masse / g $mol^{-1}$ | Dichte[1] / g $cm^{-3}$ | Viskosität[1] / mPa s | Viskosität[2] / mPa s |
|---|---|---|---|---|---|
| l-Ethyl-3-methylimidazolium bis(trifluoromethylsu1fon yl)imid | [EMIM] $[NTf_2]$ | 391,31 | 1,530 | 30,63 | 6,96 |
| l-Butyl-3-methylimidazolium bis(trifluoromethylsulfon yl)imid | [BMIM] $[NTf_2]$ | 419,36 | 1,40 | 32,46 | 7,15 |
| l-Methyl-3-octylimidazolium bis(trifluoromethylsulfon yl)imid | [OMIM] $[NTf_2]$ | 475,47 | 1,37 | 38,5 | 7,34 |
| l-Benzyl-3-methylimidazolium bis(trifluoromethylsu1fon yl)imid | [BzMIM] $[NTf_2]$ | 453,38 | 1.493 | 124,76 | 7,16 |

(fortgesetzt)

| Ionische Flüssigkeit | Abkürzung | Molar Masse / g mol$^{-1}$ | Dichte[1] / g cm$^{-3}$ | Viskosität[1] / mPa s | Viskosität[2] / mPa s |
|---|---|---|---|---|---|
| 1-Benzyl-3-ethylimidazolium bis(trif1uoromethylsulfon yl)imid | [BzEIM] [$NTf_2$] | 467,41 | 1,453 | 91,64 | 6,65 |
| 1-Benzyl-3-butylimidazolium bis(trifluoromethylsufon yl)imid | [BzBIM] [$NTf_2$] | 495,46 | 1.396 | 139,63 | 7,44 |
| 1,3-Dibenzylimidazolium bis(trifluoromethylsulfon yl)imid | [BzBzIM] [$NTf_2$] | 529,48 | | fest | 8,43 |
| | | | | [1] bei 25°C | [2] bei 100°C |

Bevorzugte Liganden, die für die Synthese von SILP-Katalysatoren eingesetzt wurden, sind zum einen der in Abbildung 4 dargestellte Diphosphit-Ligand 2,2'-(3,3'-Di-tert-butyl-5,5'-dimethoxybiphenyl-2,2'-diyl)bis(oxy)bis/4,4,5,5-tetraphenyl-1,3,2-dioxaphospholan), der aufgrund seiner Substruktur auch als Benzpinakol (BzP) bezeichnet wird.

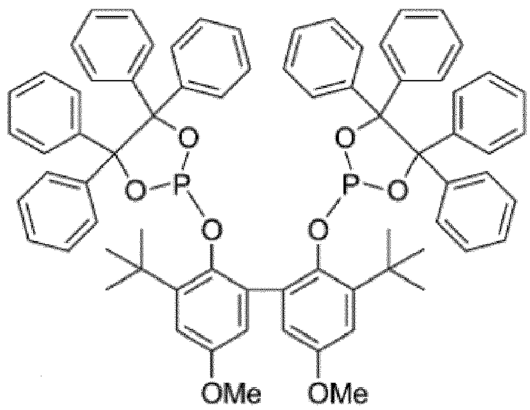

Abb. 4: Benzpinakol-Ligand

Ein weiterer bevorzugter Ligand ist der in Abbildung 5 gezeigte Ligand BIPHEPHOS (6,6'-[(3,3'-Di-tert-butyl-5,5'-dimethoxy-1,1'-biphenyl-2,2'-diyl)bis(oxy)]bis(dibenzo[d,f][1,3,2]-dioxaphosphepin)).

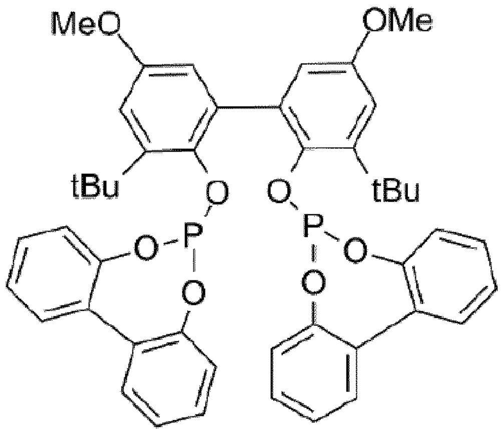

Abb. 5: BIPHEPHOS (6,6´-[(3,3´-Di-tert-butyl-5,5´-dimethoxy-1,1´-biphenyl-2,2´-diyl)bis(oxy)]bis(dibenzo[d,f][1,3,2]-dioxaphosphepin))

Als Trägermaterial für die erfindungsgemäßen SILP-Katalysatoren eignet sich u.a. Silica Gel 100, insbesondere mit einer Partikelgröße von 0,063 bis 0,2 mm. Außerdem eignen sich technische $SiO_2$ basierte Extrudate, z.B. granulierte Träger, insbesondere mit einer Partikelgröße von 1 bis 2 mm oder stäbchenförmige Träger mit beliebiger Länge und einem Durchmesser von z.B. 1 bis 2 mm, z.B. von 1,6 mm.

Ein bevorzugter Stabilisator weist die Struktur des in Abbildung 6 gezeigten sterisch gehinderten Diamins Bis(2,2,6,6-tetramethyl-4-piperidyl)sebacat auf.

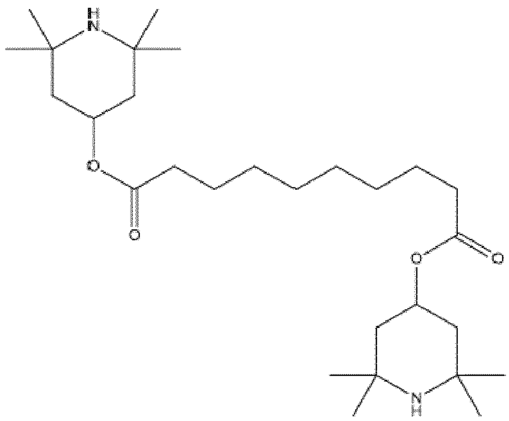

Abb. 6: Bis(2,2,6,6-tetramethyl-4-piperidyl)sebacat

Für die Herstellung erfindungsgemäßer SILP-Katalysatoren können die vorgenannten Katalysatorkomplexe, ionischen Flüssigkeiten, Liganden, Trägermaterialien und Stabilisatoren beliebig kombiniert werden.

## Patentansprüche

1. Verfahren zur Hydroformylierung von Olefinen mit Synthesegas unter Verwendung eines SILP-Katalysators, der zumindest ein kugelförmiges Katalysatorpellet mit dem Radius *R* aufweist, **dadurch gekennzeichnet, dass** das Verhältnis der Konzentration einer Komponente *i* zu deren Konzentration an der Oberfläche des SILP-Katalysatorpellets in Abhängigkeit des Ortes x durch folgende Beziehung definiert wird:

$$\frac{c_i}{c_{i,S}} = \frac{R}{x} \frac{\sinh\left(\phi_{SILP}\frac{x}{R}\right)}{\sinh(\phi_{SILP})},$$

wobei $\varphi_{SILP}$ eine dimensionslose Kennzahl für das kugelförmige Katalysatorpellet darstellt, die den Stofftransport in der Pore, über die Phasengrenzfläche zwischen Gas und ionischer Flüssigkeit und in der ionischen Flüssigkeit zur Reaktion ins Verhältnis setzt.

2. Verfahren nach Anspruch 1, **dadurch gekennzeichnet, dass** $\varphi_{SILP} = R\sqrt{\frac{\sqrt{k\, D_i^{IL}\, a}}{D_{eff}\, H_{12}}}$ ist, wobei

k = die Reaktionsgeschwindigkeitskonstante ist,
$D_i^{IL}$ = der Diffusionskoeffizient der Komponente i in der ionischen Flüssigkeit ist,
$\alpha$ = die Beladung an ionischer Flüssigkeit ist,
$D_{eff}$ = der effektive Diffusionskoeffizient ist, und
$H_{12}$ = der Henry-Koeffizient ist.

3. Verfahren nach Anspruch 1, **dadurch gekennzeichnet, dass** $\varphi_{SILP} = R\sqrt{\frac{2\, k\, \beta_i\, a}{D_{eff}\, H_{12}(k + \beta_i\, a)}}$ ist, wobei

k = die Reaktionsgeschwindigkeitskonstante ist,
$\alpha$ = die Beladung an ionischer Flüssigkeit ist,
$\beta$ = der Stoffübergangskoeffizient ist,
$D_{eff}$ = der effektive Diffusionskoeffizient ist, und
$H_{12}$ = der Henry-Koeffizient ist.

**4.** Verfahren nach Anspruch 1, **dadurch gekennzeichnet, dass** $\varphi_{SILP} = R\sqrt{\frac{k}{D_{eff}\,H_{12}}}$ ist, wobei

k = die Reaktionsgeschwindigkeitskonstante ist,
$D_{eff}$ = der effektive Diffusionskoeffizient ist, und
$H_{12}$ = der Henry-Koeffizient ist.

**5.** Verfahren nach einem der voranstehenden Ansprüche, **dadurch gekennzeichnet, dass** der Katalysatorwirkungsgrad des Katalysatorpellets durch folgende Beziehung definiert ist:

$$\eta_P = \frac{r_{eff}}{r} = \frac{3}{\phi_{SILP}}\left(\frac{1}{\tanh(\phi_{SILP})} - \frac{1}{\phi_{SILP}}\right),$$

wobei

$\eta_p$ = der SILP-Katalysatorwirkungsgrad ist,
r = die Reaktionsgeschwindigkeit und
$r_{eff}$ = die effektive Reaktionsgeschwindigkeit ist.

**6.** Verfahren nach einem der voranstehenden Ansprüche, **dadurch gekennzeichnet, dass** $\varphi_{SILP}$ kleiner als 3 ist.

**7.** SILP-Katalysator für die Hydroformylierung von Olefinen mit Synthesegas, wobei der Katalysator zumindest ein kugelförmiges Katalysatorpellet mit dem Radius *R* aufweist, **dadurch gekennzeichnet, dass** der Katalysator folgendes SILP-Modul einhält:

$$\frac{c_i}{c_{i,S}} = \frac{R}{x}\,\frac{\sinh\left(\phi_{SILP}\frac{x}{R}\right)}{\sinh(\phi_{SILP})}$$

wobei $\varphi_{SILP}$ eine dimensionslose Kennzahl für das kugelförmige Katalysatorpellet darstellt, die den Stofftransport in der Pore, über die Phasengrenzfläche zwischen Gas und ionischer Flüssigkeit und in der ionischen Flüssigkeit zur Reaktion ins Verhältnis setzt,
wobei $c_i$ die Konzentration einer Komponente i und $c_{i,s}$ deren Konzentration an der Oberfläche des SILP-Katalysatorpellets, und x der Ort ist.

**8.** SILP-Katalysator nach Anspruch 7, **dadurch gekennzeichnet, dass** $\varphi_{SILP}$ =

$$R\sqrt{\frac{\sqrt{k\,D_i^{IL}}\,a}{D_{eff}\,H_{12}}}$$

ist, wobei

k = die Reaktionsgeschwindigkeitskonstante ist,
$D_i^{IL}$ = der Diffusionskoeffizient der Komponente i in der ionischen Flüssigkeit ist,
$\alpha$ = die Beladung an ionischer Flüssigkeit ist,
$D_{eff}$ = der effektive Diffusionskoeffizient ist, und

$H_{12}$ = der Henry-Koeffizient ist.

**9.** SILP-Katalysator nach Anspruch 7, **dadurch gekennzeichnet, dass** $\varphi_{SILP} = R\sqrt{\frac{2\,k\,\beta_i\,a}{D_{eff}\,H_{12}(k+\beta_i\,a)}}$ ist, wobei

k =die Reaktionsgeschwindigkeitskonstante ist,
$\alpha$ = die Beladung an ionischer Flüssigkeit ist,
$\beta$ = der Stoffübergangskoeffizient ist,
$D_{eff}$ = der effektive Diffusionskoeffizient ist, und
$H_{12}$ = der Henry-Koeffizient ist.

**10.** SILP-Katalysator nach Anspruch 7, **dadurch gekennzeichnet, dass** $\varphi_{SILP} = R\sqrt{\frac{k}{D_{eff}\,H_{12}}}$ ist, wobei

k = die Reaktionsgeschwindigkeitskonstante ist,
$D_{eff}$ = der effektive Diffusionskoeffizient ist, und
$H_{12}$ = der Henry-Koeffizient ist.

**11.** SILP-Katalysator nach einem der voranstehenden Ansprüche, **dadurch gekennzeichnet, dass** der Katalysatorwirkungsgrad des Katalysatorpellets durch folgende Beziehung definiert ist:

$$\eta_P = \frac{r_{eff}}{r} = \frac{3}{\phi_{SILP}}\left(\frac{1}{\tanh(\phi_{SILP})} - \frac{1}{\phi_{SILP}}\right),$$

wobei

$\eta_p$ = der SILP-Katalysatorwirkungsgrad ist,
r = die Reaktionsgeschwindigkeit und
$r_{eff}$ = die effektive Reaktionsgeschwindigkeit ist.

**12.** SILP-Katalysator nach einem der voranstehenden Ansprüche, **dadurch gekennzeichnet, dass** $\varphi_{SILP}$ kleiner als 3 ist.

**13.** SILP-Katalysator nach einem der voranstehenden Ansprüche, **dadurch gekennzeichnet, dass** der Katalysator zumindest einen Liganden aufweist, der die Struktur von 2,2'-(3,3'-Di-tert-butyl-5,5'-dimethoxybiphenyl-2,2'-diyl)bis(oxy)bis/4,4,5,5-tetraphenyl-1,3,2-dioxaphospholan), auch als Benzpinakol bezeichnet, aufweist.

**14.** SILP-Katalysator nach einem der voranstehenden Ansprüche, **dadurch gekennzeichnet, dass** der Katalysator ein Übergangsmetall-Zentralatom aufweist, das ausgewählt ist aus der Gruppe, die Rhodium, Kobalt, Ruthenium, Iridium, Palladium, Platin und Eisen umfasst.

**15.** Verwendung eines SILP-Katalysators zur Hydroformylierung von Olefinen mit Synthesegas, **dadurch gekennzeichnet, dass** die Verwendung in einem Verfahren nach einem der Ansprüche 1 bis 6 erfolgt.

Fig. 1

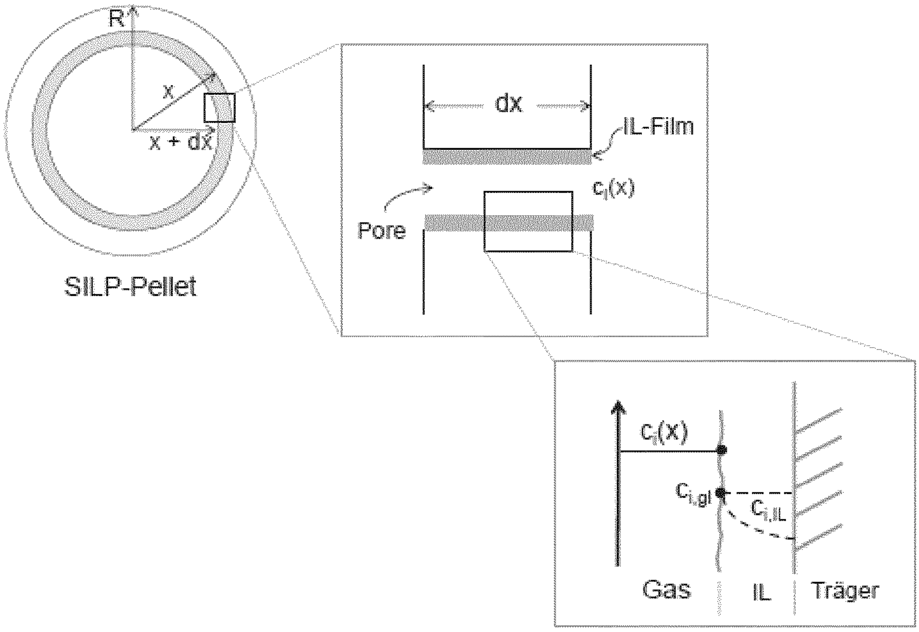


Fig. 2

| Fall I | Fall II | Fall III |
|---|---|---|
| Stofftransport und Reaktion laufen simultan im IL-Film ab | Stofftransport und Reaktion sind getrennt voneinander | Stofftransport und Reaktion sind komplett unabhängig, IL-Film ist immer gesättigt |
| 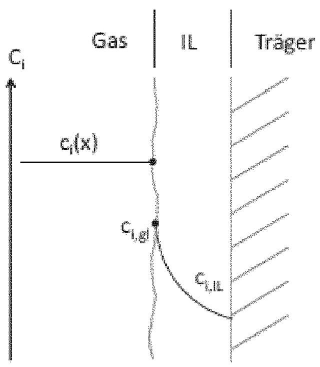  | 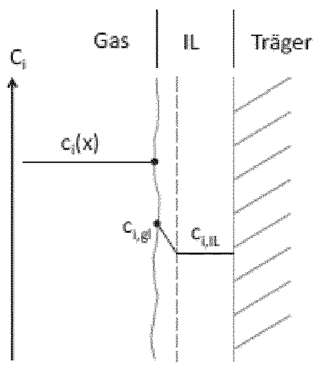  | 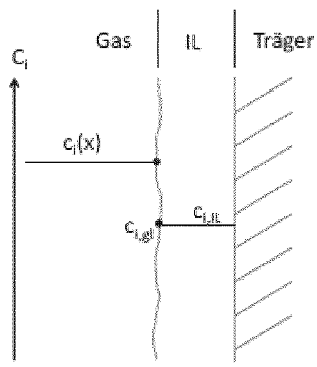  |

Fig. 3

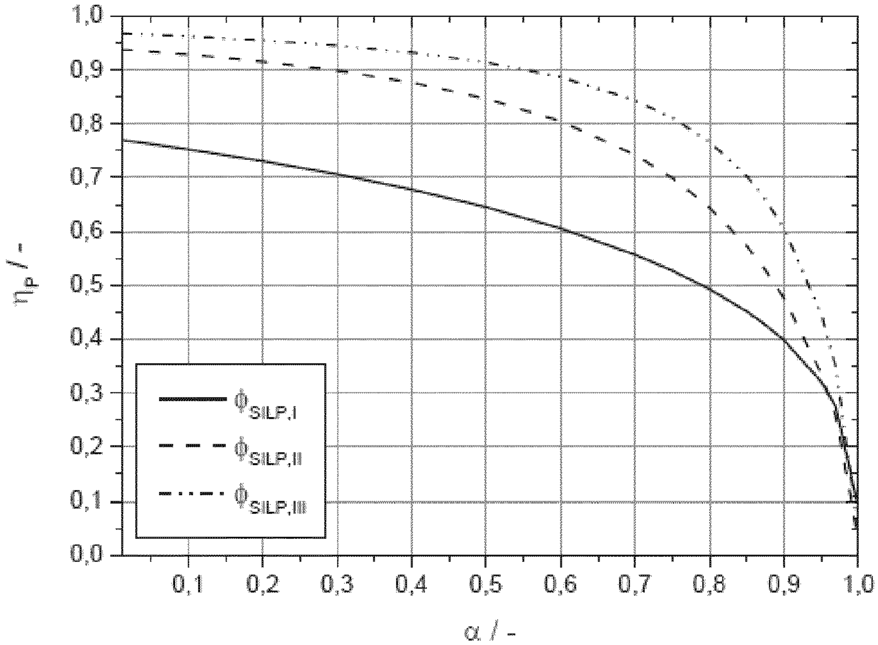


R = 0,01 m

$k = 1\ s^{-1}$

$D_{eff} = 1 \cdot 10^{-5}\ m^2\ s^{-1}$

$D_{i,IL} = 1 \cdot 10^{-10}\ m^2\ s^{-1}$

$\varepsilon = 0,6$

$H_{12} = 50$

Fig. 4

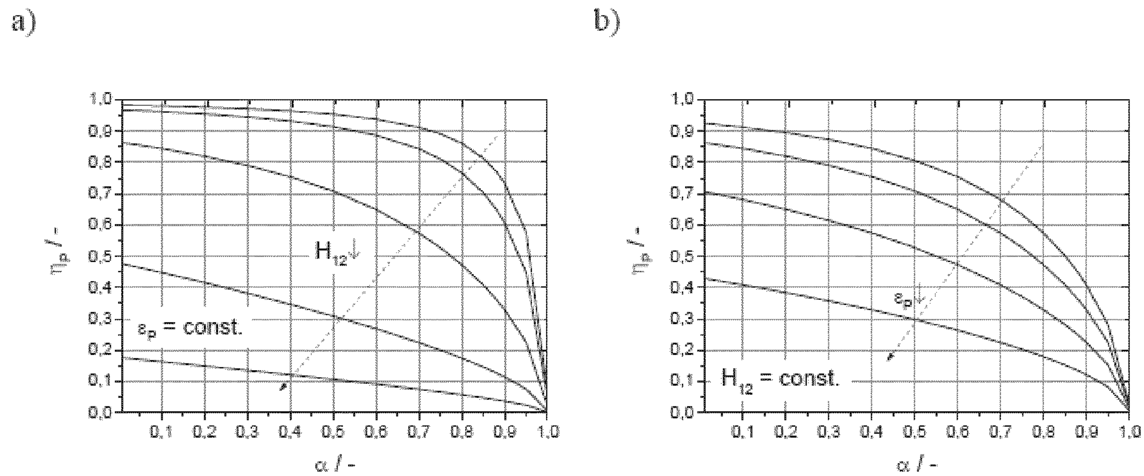

Fig. 5

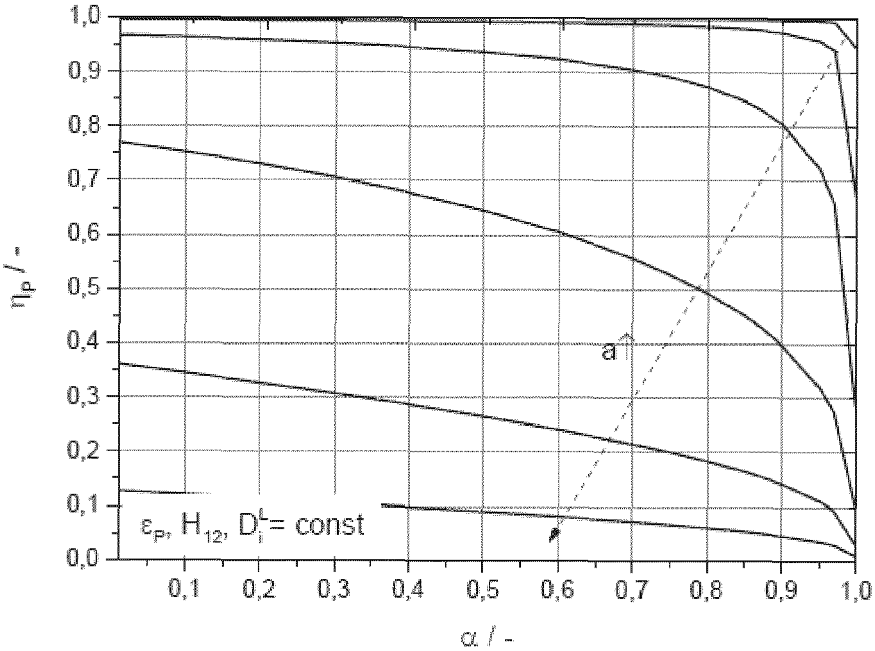
1,0
0,9
0,8
0,7
0,6
0,5
0,4
0,3
0,2
0,1
0,0
η_P / -
a
ε_P, H_12, D_i^L = const
0,1 0,2 0,3 0,4 0,5 0,6 0,7 0,8 0,9 1,0
α / -

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **E. LINDNER ; T. SCHNELLER ; F. AUER ; H. A. MAYER.** Chemistry in Interphases- A New Approach to Organometallic Syntheses and Catalysis. *Angew. Chem., Int. Ed.,* 1999, vol. 38, 2154-2174
- **D. J. COLE-HAMILTON ; R. P. TOOZE.** Catalyst Separation, Recovery and Recyling. Springer, 2006
- **E. W. THIELE.** Relation between Catalytic Activity and Size of Particle. *Ind. Eng. Chem.,* 1939, vol. 31, 916-920
- **W. K. LEWIS ; W. G. WHITMAN.** Principles of Gas Absorption. *Ind. Eng. Chem.,* 1924, vol. 16, 1215-1220
- **M. SOBOTA ; I. NIKIFORIDIS ; W. HIERINGER ; N. PAAPE ; M. HAPPEL ; H.-P. STEINRUCK ; A. GORLING ; P. WASSERSCHEID ; M. LAURIN ; J. R. LIBUDA.** Toward Ionic-Liquid-Based Model Catalysis: Growth, Orientation, Conformation, and Interaction Mechanism of the [Tf2N]-Anion in [BMIM][Tf2N] Thin Films on a Well-Ordered Alumina Surface. *Langmuir,* 2010, vol. 26, 7199-7207
- **M. HAUMANN ; A. SCHÖNWEIZ ; H. BREITZKE ; G. BUNTKOWSKY ; S. WERNER ; N. SZESNI.** Solid-State NMR Investigations of Supported Ionic Liquid Phase Water-Gas Shift Catalysts: Ionic Liquid Film Distribution vs. Catalyst Performance. *Chem. Eng. Technol.,* 2012, vol. 35, 1421-1426